# EUROPEAN PATENT APPLICATION

(11) **EP 1 062 867 A1**
(43) Date of publication of application: **27.12.2000**
(21) Application number: 99201976.0
(22) Date of filing: 21.06.1999
(51) Int. Cl.: A01H 5/08

(54) **Strawberry derived flavours, strawberries, strawberry plants and methods of producing such aswell as application of the mentioned products**

(71) Applicant: QUEST INTERNATIONAL B.V., 1411 GP Naarden (NL)
(72) Inventor: Weenen, Hugo, 1261 RV Blaricum (NL); Glasius, Martin Eduard, 3766 BG Soest (NL); Bruinje, Arnold, 1272 CG Huizen (NL); Stam, Arjan Cornelis, 2264 LJ Leidschendam (NL); Bouter, Nico, 1261 LK Blaricum (NL); Jägers, Paul Paulines Joseph Maria, 3765 WJ Soest (NL); Kerler, Josef, 1411 PD Naarden (NL); Scanlan, Francis Patrick, 1017 WC Amsterdam (NL)
(74) Representative: de Bruijn, Leendert C.

(57) **Abstract**

The invention is directed at flavour impact components derived from strawberries. Suitably these can be used in foodstuff preparation. The advantage is that they are derived from their natural source. The invention also covers novel strawberries and plants capable of producing such. The invention also covers application of the strawberries and derivatives thereof in foodstuffs requiring strawberry flavour. The flavour impact component can also be removed from the srawberries and application of the flavour impact components in particular HDF and GDL are illustrated.

## Description

### Summary of the invention.

The subject invention is directed at flavour building blocks derived from strawberry. Suitably these building blocks can be used in foodstuff preparation. The advantage is that they are derived from or are present in the natural source.

The improvement according to the invention renders it possible to use a reduced amount of fruit or to obtain the same amount of flavour as was possible with the prior art fruit without requiring the addition of additional flavour. The improvement also provides naturally strawberry derived flavour building blocks either as such or contained in a fruitprep or extract i.e. a flavour building block being isolated from the strawberry or being present in a composition in a strawberry derived food product or strawberry derived ingredient mixture such as an extract or fruitprep. Suitably the flavour building block is used in foodstuffs in general. Quite suitably they can be used for preparing fruitpreps e.g. for preservatives,jams syrup concentrates or for dairy products requiring strawberry flavour. The invention covers foodstuffs comprising the natural flavour building blocks.

The invention also covers strawberries as such in which the flavour building blocks are present in extremely high amounts far exceeding those of prior art strawberries. The invention is also directed at plants for producing such strawberries. The invention also covers the products comprising the flavoring compounds derived from the strawberries or the strawberries as such according to the invention.

### Introduction and background to the invention.

In the USA the term natural fruit flavour or fruit extract is based on preparations that are made entirely from the corresponding fruit. Delivering natural strawberry flavours to the consumer market in particular the US market is quite challenging. It excludes the use of cell suspension cultures as well as the addition of precursors. In particular the problem of developing strawberry derived flavour building blocks is that strawberries are not sufficiently rich in flavour volatiles to allow economical production of natural strawberry flavours by extracting or isolating the key flavour compounds.

The subject invention is directed at providing a solution to this problem.

### Detailed description to the invention.

The subject invention is directed in particular at the development of 7 specific key substances described as sweet, lactony, fruity, green, acidic, sulphury and floral Specifically interesting blocks are the blocks sweet and lactony. Two key substances 4-hydroxy-2,5-dimethyl-3(2H)-furanone (=HDF) and gamma-decalactone (=GDL) were selected as representative substances for the aforementioned flavour building blocks.

Strawberry plants providing fruit comprising much higher amounts of character impact compounds were developed. The goal was to develop several natural building blocks enriched in specific groups of metabolically related flavour substances through plant breeding of strawberry cultivars followed by the use of the building blocks to create a well balanced strawberry derived flavour.

The project strategy was as follows:

Definition of the desired target building blocks and definition of the key aroma compounds was required. A number of key substances were defined and described which included i.a. as sweet on the one hand and lactony on the other. The two key substances 4-hydroxy-2,5-dimethyl-3(2H)-furanone (=HDF) and gamma-decalactone (=GDL) were selected as mainly responsible for the sweet respectively lactony flavour impression. A convenient and reproducible method of quantitative analysis was developed and strawberry cultivars had to be screened for the key aroma compounds. Subsequently the best cultivars needed to be selected and crosses were made with the selected cultivars.

The daughters of these crosses needed to undergo analysis also in order to select a daughter bearing fruit with the desired increased amount of GDL and/or HDF. Subsequently the strawberries were tested in application. The strawberry derived flavour building blocks could be used either directly through introduction of strawberry from the selected daughter or through isolation of flavour e.g. from liquefied strawberries. Alternatively the daughter cultivar or cultivars can be crossbred further and their offspring selected for the desired key compound in higher amounts than the strawberries resulting from the preceeding breeding cycle. Naturally this can be carried out through a number of selection cycles but it is preferable to use the first generation with a view to obtaining the flavour in an increased amount as soon as possible. It is possible to use liquefied strawberries as source of the desired flavour compounds, or a concentrate obtained through freeze-drying. Alternative methods can be used by a person skilled in the art of extracting flavour or processing fruit.

Plant breeding of 8 selected parent cultivars resulted in very high levels of HDF and GDL in the daughter cultivars. The improvement with factors as high as 3-5 times as high as the initial value in parents with already high amounts of HDF and GDL was unexpected. This was remarkably achieved in one crossing. In fact values up to 6 -7 times the height of the concentration present in the parents have been reached. The invention thus covers strawberries having either a concentration in ppm of HDF of at least 60 ppm and/or a concentration of GDL of at least 10 ppm. The strawberries according to the invention are preferably naturally derived i.e. not genetically modified by recombinant DNA technology or treatment with at least one mutagen. The strawberries according to the invention are arrived at preferably by cross breeding cultivars exhibiting levels of HDF of at least 15 ppm or levels of GDL of at least 2.5 ppm. The levels of the flavour compounds are measured when the strawberry is red i.e. between orange and dark red. The levels are measured when the strawberry is considered ripe but not after they have reached the stage known as overripe.

Suitably the parents are selected exhibiting as high a level of either HDF or GDL as possible. In the examples parents with levels of HDF of 7-45 ppm were used. Levels of GDL of 0-5,8 ppm were used. These parents are illustrative of the highest levels of HDF and GDL found in prior art cultivar strawberries. The strawberries according to the invention preferably exhibit at least double the level of the parent's levels of HDL and/or GDF. Suitably the level of increase is even higher e.g. 3, 4 or 5 times higher for one or both of HDF and GDL. In the examples we illustrate even levels of 6-7 times increase. Preferably even higher than 5 times the level of the parents' HDF and/GDL level is achieved. Most preferable is an increase in both components levels. Strawberries according to the invention can be achieved of more than 80 ppm, even more than 100 ppm and also more than 120 ppm HDF. The Example shows achievement of strawberries with 32, 57, 104, 108 and 147 ppm HDF. Strawberries according to the invention comprising more than 10 ppm GDL,.even more than 15 ppm GDL and even more than 25 and 30 ppm GDL have been achieved. The Example shows achievement of strawberries with 20, 27 and 32 ppm GDL.

The strawberries according to the invention exhibiting the increased levels of HDF and/or GDL surprisingly still taste good as such. This is quite surprising considering the extreme degree in which the levels of flavour components have changed. The strawberry plants also do not exhibit any phenotypically discernible negative effects of the increase in HDF and/or GDL levels. The strawberry plants according to the invention are also able to reproduce so that the cultivar can be maintained without requiring anything other than natural reproduction route.

The strawberries according to the invention have also been subjected to processing steps that strawberries currently undergo when being used as additives for foodstuffs. For example fruitpreps have been made for use in preservatives, jams, syrup concentrates and dairy products. The amounts of strawberry required to develop good flavour for these applications are lower than previously required for prior art fruitpreps. The strawberries according to the invention provide sufficient flavour per kg of strawberry to provide good flavouring in cases where equivalent amounts of fruit of the prior art required addition of additional flavouring components. The invention now renders it possible to develop products with solely natural strawberry derived flavouring.

Strawberry plants achieved without genetic engineering (i.e. achieved through breeding) that produce strawberries as defined above according to the invention are also included within the scope of the invention. It is pointed out that to arrive at such plants a first selection of the parents based on measurement of the desired ehanced flavour impact component e.g. HDF and/or GDL level must occur followed by natural reproduction and subsequently the progeny must also undergo the measurement of the resulting progeny in order to arrive at a cultivar according to the invention capable of producing a strawberry according to the invention. As can be seen from the population density curves presented in the figures illustrating the HDF or GDL content of the parent populations and the resulting daughter population the population density curve has moved significantly to the right to higher contents of HDF and/or GDL. This happens for anty of the flavour impact components. Plants from the right side of the population density curve obviously are preferred as these have the higher concentration of the flavour impact component e.g. HDF and/or GDL. Preferably plants illustrating higher component levels for more than one flavour impact component e.g. for both HDF and GDL are preferred. Clearly technical means of measuring the levels of the desired flavour impact component e.g. either HDF and/or GDL are essential to achieving the end result. The measurements are required to select the best progeny both at the parent level and at the daughter level.

The invention thus also covers a method of arriving at plants and subsequently strawberries according to the invention said method comprising the steps of selecting parent strawberries or strawberry cultivars producing strawberries exhibiting levels of HDF and/or GDL higher than 60 and 10 ppm respectively, followed by generating progeny from the natural reproduction process of the selected parents, said method further comprising determining the level of the HDF and/or GDL in the strawberries of the resulting progeny and selecting a strawberry cultivar with strawberries exhibiting more than 60 ppm HDF and/or more than 10 ppm GDL.. Levels of more than 15 even more than 20 , more than 25 and even more than 30 ppm GDL are preferred. For HDF the range can go from 60 ppm higher than 80 ppm even higher than 100 ppm.

In a preferred embodiment the selection criteria for levels of HDF and/or GDL correspond to those given already above for strawberries according to the invention. If the parents are already themselves strawberries or strawberry cultivars according to the invention then any progeny resulting therefrom is also part of the invention. The invention also covers any application of strawberries according to the invention. This covers application as such as foodstuff or in foodstuff, reproduction and use thereof for generating flavour in foodstuffs in any way that results in the presence of HDF and/or GDL derived from the strawberry. Naturally the foodstuffs and any precursors comprising HDF and/or GDL of the strawberries according to the invention are covered by the invention. Any HDF and/or GDL obtained from a strawberry according to the invention and any product comprising such HDF and/or HDL according to the invention is included within the scope of the protection. In particular dairy products and preservatives ,jams, syrup concentrates and beverages or intermediate products i.e. between the raw materials and the final products exhibiting strawberry flavour are included.

The invention will be illustrated in further detail in the following text. and in particular in the claims. The method for measuring the level of flavour impact component can be derived from the prior art and will be clear to the person skilled in the art in addition the method for measuring HDF or GDL is described in the analysis below.

### Analysis

Strawberries (50 g) were cut into pieces and extracted with diethylether (50 ml) for 2h, containing naphthalene as internal standard. The organic phase was separated and dried over sodium sulphate. 1 ml of extract and 0.1 ml of a mixture of hexamethyldisilazane and trimethylchlorosilane (2:1, v/v) were put in a vial, reacted for 0.5 h and analysed by gc/ms. GC parameters: column RTX-50 (30m x 0.25mm i.d. x 0.50µm); temperature program: 150°C/lmin. - 20°C/min. - 280°C/2.5min. MS parameters: SIM mode; selected ions m/z: trimethylsilyl-HDF 85 and 200, GDL 85 and 128, naphthalene 128.

Standard regression curves were drawn for HDF and GDL and their recoveries were determined by standard addition of two analytes to a blank strawberry pulp (HDF 81% and GDL 76%).

### Results

For the creation of novel strawberry derived flavours, 7 target building blocks were defined, which are characterised by 10 key aroma compounds (Table 1).

**Table 1.**

| Target building blocks and key aroma compounds | |
|---|---|
| building block | characterised by |
| sweet | 4-hydroxy-2,5-dimethyl-3(2H)-furanone (HDF) |
| lactony | γ-decalactone (GDL) |
| fruity | methyl butanoate, ethyl butanoate |
| green | (E)-2-hexenal, hexanal |
| acidic | 2-methylbutanoic acid, 3-methylbutanoic acid |
| sulphury | methyl thioacetate |
| floral | linalool |

Daughter plants resulting from crossing of selected strawberry cultivars can be expected to be enhanced in metabolically related flavour substances. We elected to start with the two key blocks 'sweet' and 'lactony', which are characterised by 4-hydroxy-2,5-dimethyl-3(2H)-furanone (HDF) and γ-decalactone (GDL).

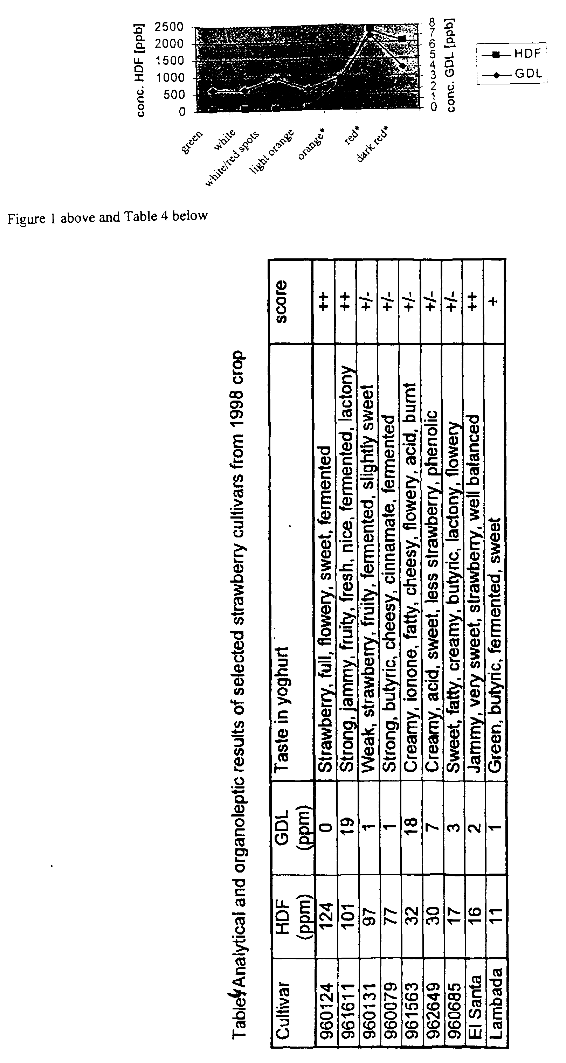

A plant-breeding institute (CPRO-DLO, Wageningen, The Netherlands) carried out crossings and harvesting of strawberries. In order to define the optimal harvesting stage of strawberries, the formation of HDF and GDL was followed analytically during 7 stages of ripening. As illustrated in Figure 1, both compounds HDF and GDL showed highest concentrations, when strawberries were 'red' (mature).

Screening of more than 220 different strawberry cultivars resulted in a selection of 8 cultivars including the commercially well known Senga Precosa. They showed the highest HDF and/or GDL concentrations. All cultivars were also screened for the other previously mentioned key compounds (see Table 1, data not shown), in order to be able to make a selection for other building blocks as well.
6500 daughter plants were obtained by 26 crosses (in 1994), 4479 of which were analysed for HDF and GDL (in 1996). Quantitative analysis was performed by gc/ms using an internal standard. This was repeated for the 48 best cultivars in 1997.

Figure 1. shows concentrations of 4-hydroxy-2,5-dimethyl-3(2H)-furanone (HDF) and γ-decalactone (GDL) with respect to stages of strawberry ripening

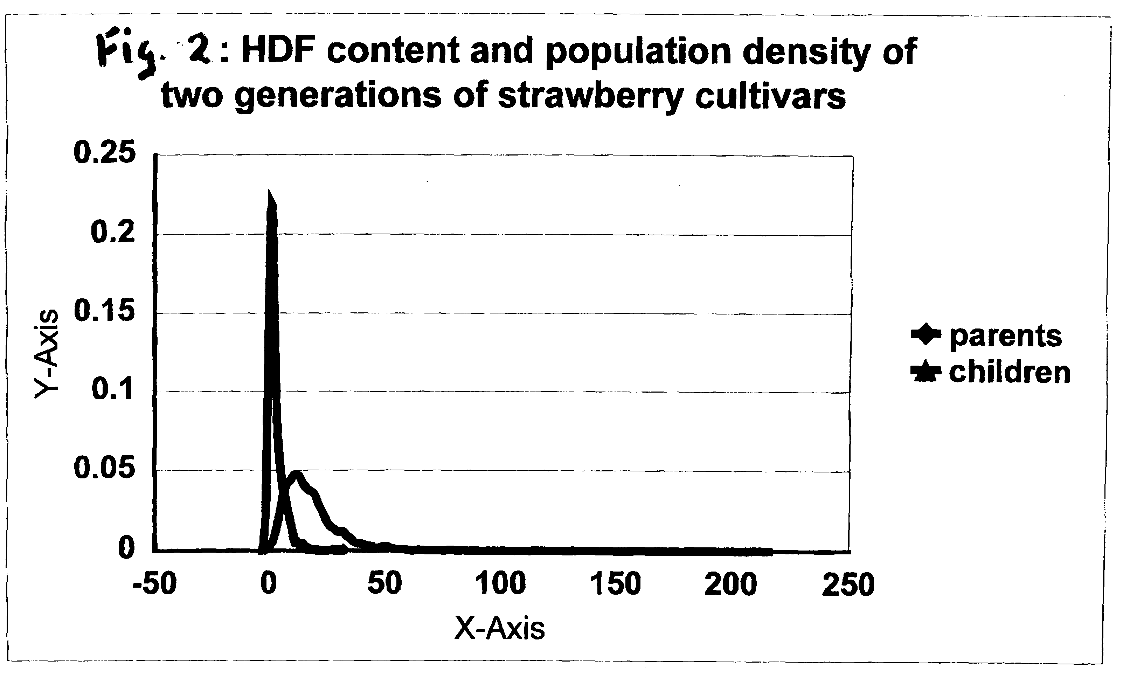

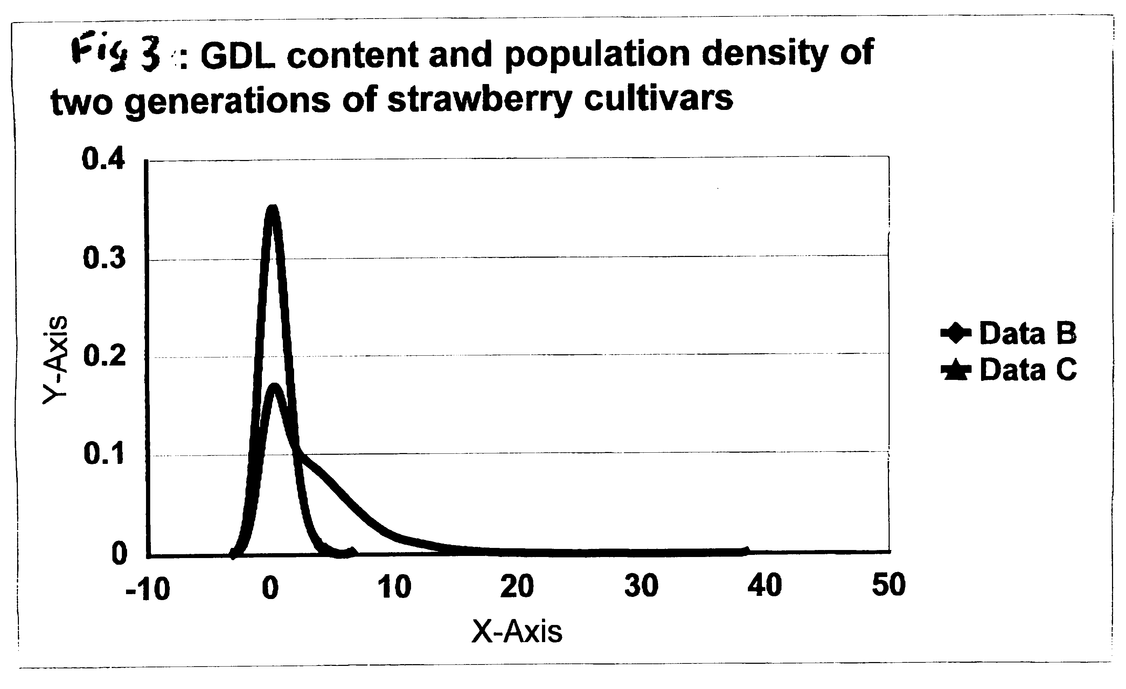

In Figures 2 and 3 the HDF and GDL contents of both parent and daughter populations are visualised by means of density estimation charts.
Table 2 shows the analytical data of 5 daughter plants with the highest concentrations of HDF (no.1-3) and GDL (no.3-5) as well as the data of the corresponding parents.

**Table 2.**

| Concentrations of 4-hydroxy-2,5-dimethyl-3(2H)-furanone (HDF) and γ-decalactone (GDL) in parents and daughter plant of the 5 best cultivars^{a} | | | | | | |
|---|---|---|---|---|---|---|
| No. | conc. of HDF [ppm] in | | factor | conc. of GDL [ppm] in | | factor |
| | parents(b) | daughter© | | parents(b) | daughter© | |
| 1 | 23 | 147 | 6.4 | 3.6 | 0 | 0 |
| 2 | 20 | 108 | 5.4 | 2.4 | 0 | 0 |
| 3 | 36 | 104 | 2.9 | 5.3 | 32 | 6.0 |
| 4 | 36 | 32 | 0.9 | 5.3 | 27 | 5.1 |
| 5 | 26 | 57 | 2.2 | 2.9 | 20 | 6.9 |

| | | | | | | |
|---|---|---|---|---|---|---|
| ^{a}: The best cultivars are those which show the highest concentrations of HDF and/or GDL. | | | | | | |
| ^{b}: The concentrations are mean values of the two parents. | | | | | | |
| ^{c}: Mean values of duplicates (from 1996 and 1997). | | | | | | |

The highest HDF level detected in daughter plants was 147 ppm (no.1), which is an increase in concentration by a factor of 6.4. Concerning GDL levels, cultivars no. 3-5 were increased by factors between 5-7, with the highest concentration reaching 32 ppm (no.3).

In 1998, fruit preparations (strawberry jams) were prepared from the 10 best cultivars and evaluated organoleptically. In Table 3, the odour activity values of HDF and GDL in jams of the best two strawberry cultivars were compared to those of the commercially available cultivar 'Lambada'. The analytical data are also compared with the results of organoleptic evaluations. The fruit preparations of cultivar no. 2 and 3 showed very good organoleptic properties in yoghurt, which is probably, based on the high odour activity values of HDF. The high GDL level of cultivar no.3 is responsible for the lactony character of this building block. These two products are the first two unique, strawberry derived building blocks that were made available in our study. In Table 4 the analytical and organoleptical results of selected strawberry cultivars from the 1998 crop are provided. The taste was determined in yoghurt and the flavour impact component was added as a fruitprep containing 50% fruit in a dosage of 15%.

**Table 3.**

| Odour activity values of HDF and GDL, aroma description/scores of fruit preparations of the best two strawberry cultivars obtained by crossbreeding and of a commercial cultivar. | | | | |
|---|---|---|---|---|
| Cultivars^{b} | odour activity value^{a} | | aroma description^{d} | score^{e} |
| | HDF | GDL | | |
| no.2 | 10800 | < 1 | strawberry-like, full, flowery, sweet, fermented, fruity | ++ |
| no.3 | 10400 | 6400 | strawberry-like, fruity, fresh, lactony, fermented | ++ |
| Lambada^{c} | 400 | 420 | green, fermented, sweet | +/- |

| | | | | |
|---|---|---|---|---|
| ^{a}: Odour activity values (= concentration divided by odour thresholds) are based on odour thresholds in water, according to (3) and (4). | | | | |
| ^{b}: The cultivars were obtained by crossbreeding; the numbering refers to Table 1. | | | | |
| ^{c}: Lambada is a commercially available cultivar. | | | | |
| ^{d}: The aroma of the fruit preparations was evaluated on yoghurt by our flavorists. | | | | |
| ^{e}: Aroma scores: +/- reasonable; + good; ++ very good. | | | | |

Although other building blocks have to be developed, these two blocks can already be used as fully strawberry derived flavourings for yoghurt and ice cream by way of example.

Thus the principle of enriching flavour substances through plant breeding has been demonstrated. In one breeding cycle a 6-7 times enrichment of HDF and GDL levels was achieved, resulting in strawberry derived building blocks with added flavour value.

### References

1. H.W.C. Raaijmakers, F.G.J. Voragen, G. Beldman and H.A. Schols. Personal communication (1995).
2. R. Roscher, G. Bringmann, P. Schreier and W. Schwab. Radiotracer studies on the formation of 2,5-dimethyl-4-hydroxy-3(2H)-furanone in detached ripening strawberry fruits. J. Agric. Food Chem. 46:1488-1493 (1998).
3. T. Hofmann and P. Schieberle. Evaluation of character impact odorants in fresh strawberry juice by quantitative measurements and sensory studies on model mixtures. J. Agric. Food Chem. 45: 227-232 (1997).
4. M. Larsen and L. Poll. Odour thresholds of some important aroma compounds in strawberries. Z. Lebensm. Unters. Forsch. 195: 120-123 (1992).

## Claims

1. A strawberry exhibiting significantly enhanced flavour profile, said strawberry being obtained through classical breeding using parents high in at least one flavour impact component and the strawberry being selected by measuring for the enhanced concentration of a flavour impact component representative for any of the following building blocks sweet, lactony, fruity, green, acidic, sulphury and floral as e.g. are characterised by. 4-hydroxy-2,5-dimethyl-3(2H)-furanone (HDF) γ-decalactone (GDL) methyl butanoate, ethyl butanoate(E)-2-hexenal, hexanal, 2-methylbutanoic acid, 3-methylbutanoic acid, methyl thioacetate and linalool, preferably sweet and lactony as represented for example by HDF and GDL and said strawberry preferably further exhibiting a significantly enhanced general strawberry profile.

2. A strawberry according to claim 1 having either a concentration in ppm of HDF of at least 60 ppm and/or a concentration of GDL of at least 10 ppm, suitably exhibiting a concentration in ppm of HDF of at least 80 ppm, preferably, even more than 100 and even more than 120 ppm, also suitably more than 130 ppm HDF., suitably more than ,140 ppm and/or suitably having a concentration in ppm of GDL of at least 15 ppm GDL, even more than 20 ppm GDL and even more 25 and 30 ppm GDL.

3. A strawberry derived through classical breeding exhibiting a concentration in ppm of flavour impact component wherein at least one of said components, preferably HDF and/or GDL exhibits a concentration more than double that of strawberries of the parent plants, preferably more than 3 fold, 4fold, 5fold,6 fold or 7fold, preferably being a strawberry according to any of the preceeding claims.

4. A strawberry according to any of the preceeding claims, said strawberry being naturally derived i.e. not being genetically modified by recombinant DNA technology or treatment with any mutagen.

5. A strawberry according to any of the preceeding claims, said strawberry not exhibiting any phenotypically discernible negative effects of the increase in flavour impact component for example the increase of HDF and/or GDL levels.

6. A strawberry according to any of the preceeding claims, said strawberry being able to reproduce so that the resulting cultivar can be maintained without requiring anything other than a natural reproduction route.

7. A method for developing strawberries exhibiting significantly enhanced flavour profile obtained by classical breeding using parents high in at least one of a limited number of flavour impact components and selecting the desired strawberry by measurement of a compound representative for the flavour type of choice

8. A method according to claim 7 wherein the flavour component representative for any of the following building blocks sweet, lactony, fruity, green, acidic, sulphury and floral as e.g. are characterised by. 4-hydroxy-2,5-dimethyl-3(2H)-furanone (HDF) γ-decalactone (GDL) methyl butanoate, ethyl butanoate(E)-2-hexenal, hexanal, 2-methylbutanoic acid, 3-methylbutanoic acid, methyl thioacetate and linalool., preferably sweet and lactony as represented for example by HDF and GDL

9. A method for producing a strawberry according to any of the preceeding claims 1-6 and/or a strawberry plant according to claim 13 by cross breeding cultivars bearing strawberries exhibiting concentrations of HDF of at least 15 ppm and/or levels of GDL of at least 2,5 ppm, followed by the step of harvesting daughter strawberries borne by a resulting daughter strawberry cultivar, said daughter strawberry having either a concentration in ppm of HDF of at least 60 ppm and/or a concentration of GDL of at least 10 ppm. and/or said daughter strawberry exhibiting a concentration in ppm of HDF and/or GDL more than double that of strawberries of the parent plants, preferably more than 3 fold, 4fold, 5fold, 6 fold or 7fold.

10. A method according to the preceeding claim wherein at least one measurement of the flavour impact component concentration e.g. HDF and/or GDL concentration in a strawberry occurs, wherein said method comprises a first selection for parent strawberry cultivars with strawberries exhibiting the required minimum concentration of the desired flavour impact component e.g.HDF and/or GDL ,said selection occuring optionally by measurement of the the desired flavour impact component concentration e.g. HDF and/or GDL concentration or by virtue of information available on the concentrations readily available from a previous measurement, followed by a step of natural reproduction using the selected parent cultivars to produce a daughter cultivar, growing said resulting cultivar under conditions resulting in strawberry production, said step being followed by measurement of fruit borne by the daughter cultivar for determining the concentration of the desired flavour impact component concentration e.g. HDF and/or GDL and subsequently upon measuring the presence of the desired concentration selecting the daughter plant and any strawberry derived from it or a descendant thereof for production of the required strawberries

11. A method according to any of claims 7-10 wherein the selection criteria for levels of flavour impact components e.g. HDF and/or GDL in the daughter cultivars strawberries correspond to those given already above for strawberries according to any of claims 1-6

12. A method according to any of the claims 7-11, wherein a further selection step for the daughter strawberry is an organoleptic test..

13. Strawberry plants achieved without genetic engineering i.e. achieved through breeding said strawberry plants capable of producing strawberries as defined above in any of claims 1-6., in particular strawberry plants capable of bearing strawberries according to any of claims 1-6, said strawberry plant being obtained, by any of the methods of claims 7-12 or said strawberry plant being obtained by a natural reproduction process, said natural reproduction process using at least one parent defined as being either a strawberry according to any of claims 1-6 or said parent being a cultivar bearing strawberries according to any of claims 1-6., preferably in the latter case both parents in the reproduction process are as defined for the at least one parent.

14. A strawberry flavoured product comprising a strawberry according to any of claims 1-6 or a strawberry derived according to the method of any of claims 7-12 or derived from a strawberry plant according to claim 13 to the product or an ingredient to be used in the product, whereby the strawberry as such is added, whereby the product preferably is a foodstuff or ingredient for addition to a foodstuff.

15. A strawberry flavoured product comprising a flavour impact component as mentioned in claim 1,(e.g.HDF and/or GDL) obtained from or being present in a derivative of a strawberry according to any of claims 1-6 or a strawberry derived according to the method of any of claims 7-12 or a strawberry derived from a strawberry plant according to claim 13 whereby the product preferably is a foodstuff or ingredient for addition to a foodstuff.

16. A strawberry flavoured product according to claim 14 or 15 wherein the flavour impact component as mentioned in claim 1,(e.g the HDF and/or GDL) has been isolated from the strawberry

17. A strawberry flavoured product according to any of the claims 13-16 not being a strawberry but e.g. being a liquid, a gel, a solid or a powder more explicitly by way of example being a fruitprep,a beverage, a preservative, a jam, a syrup concentrate or a dairy product.

18. A strawberry flavoured product exhibiting good strawberry flavour requiring less strawberry than a corresponding product comprising prior art strawberries and/or a product in which the strawberries according to the invention provide sufficient flavour per kg of strawberry to provide good flavouring in cases where equivalent amounts of fruit of the prior art required addition of additional flavouring components.

19. Flavour impact compound derived from a strawberry according to any of claims 1-6, preferably a flavour impact component as defined in any of claims 1-6.
